# EUROPÄISCHE PATENTSCHRIFT

(11) **EP 3 080 066 B1**
(45) Veröffentlichungstag und Bekanntmachung des Hinweises auf die Patenterteilung: **21.02.2018**
(21) Anmeldenummer: 14809847.8
(22) Anmeldetag: 10.12.2014
(51) Int. Cl.: C07C 45/50, B01J 31/02, B01J 31/18, B01D 61/02

(54) **ZWEISTUFIGE HYDROFORMYLIERUNG MIT KREISGAS- UND SILP-TECHNOLOGIE**
TWO-STEP HYDROFORMYLATION WITH GAS-RECYCLE AND SILP-TECHNOLOGY
HYDROFORMYLATION EN DEUX ETAPES AU MOYEN DES TECHNOLOGIES DE CIRCULATION DE GAZ ET DE PHASE IONIQUE LIQUIDE SUPPORTÉE (SILP)

(30) Priorität: 13.12.2013 DE 102013225883
(43) Veröffentlichungstag der Anmeldung: 19.10.2016
(73) Patentinhaber: Evonik Degussa GmbH, 45128 Essen (DE)
(72) Erfinder: BECKER, Marc, 44359 Dortmund (DE); DYBALLA, Katrin Marie, 45657 Recklinghausen (DE); FRANKE, Robert, 45772 Marl (DE); FRIDAG, Dirk, 45721 Haltern am See (DE); HAHN, Hanna, 47199 Duisburg-Baerl (DE); LAZAR, Marina, 63505 Langenselbold (DE); PRISKE, Markus, 5020 Salzburg (AT); STOCHNIOL, Guido, 45721 Haltern am See (DE)
(86) Internationale Anmeldenummer: PCT/EP2014/077110
(87) Internationale Veröffentlichungsnummer: WO 2015/086634

(56) Entgegenhaltungen:
- EP-B1- 2 280 920
- WO-A1-2012/041846
- DE-A1- 10 128 325
- US-A- 4 593 127
- US-B1- 6 414 202

## Beschreibung

Die Erfindung betrifft Verfahren zur Herstellung von Aldehyden durch Hydroformylierung von Alkenen, bei welchem ein alkenhaltiges Einsatzgemisch mit Synthesegas in Gegenwart eines homogenen Katalysatorsystems einer primären Hydroformylierung unterworfen wird, wobei die primäre Hydroformylierung in einer primären Reaktionszone erfolgt, aus welcher kontinuierlich ein Kreisgas enthaltend zumindest ein Teil der Produkte, sowie nicht umgesetzte Edukte der primären Hydroformylierung abgezogen und teilweise kondensiert wird, wobei nicht kondensierte Anteile des Kreisgases in die primäre Reaktionszone zurückgeführt werden, und wobei kondensierte Anteile des Kreisgases in einer Aldehydabtrennung destillativ aufgetrennt werden in ein aldehydreiches Gemisch und in ein aldehydarmes Gemisch.

Ferner betrifft die Erfindung Anlagen, Anlagenkomplexe und deren Verwendung zur Durchführung dieser Verfahren.

In der organischen Chemie werden Stoffgruppen gemeinhin nach der Anzahl ihrer Kohlenstoffatome eingeteilt. Dabei wird der interessierenden Stoffklasse das Präfix Cₙ-vorangestellt, wobei n für die Anzahl der jeweiligen Kohlenstoffatome des Stoffes steht. Ist beispielsweise von C₄-Alkenen die Rede, so sind die vier isomeren Olefine mit vier Kohlenstoffatomen gemeint, nämlich Isobuten, 1-Buten, cis-2-Buten und trans-2-Buten. Demgegenüber existiert nur ein Alken mit drei Kohlenstoffatomen, nämlich Propen und ein C₃-Alkan, nämlich Propan.

Die gesättigten Alkane sind kaum reaktiv und werden deswegen überwiegend als Brennstoff oder Aerosol-Treibstoff verwendet.

Aus den reaktiveren Alkenen lassen sich indes Kohlenwasserstoffe mit einer größeren Anzahl an Kohlenstoffatomen aufbauen, die ein weites Anwendungsspektrum erschließen und damit höhere Verkaufspreise erzielen als die Ausgangsstoffe mit einer geringen Anzahl Kohlenstoffatome. Auf diese Weise schöpft die industrielle organische Chemie Wert.

Eine wirtschaftlich bedeutende Stoffklasse, die aus dieser Motivation aus niederen Alkenen herstellt wird, sind die Aldehyde.

Zu den Aldehyden mit vier Kohlenstoffatomen gehören die isomeren Stoffe n-Butanal und iso-Butanal. Sie werden als für die Herstellung von Vulkanisationsbeschleuniger, Kunstharzen und Weichmacher weltweit in großen Mengen nachgefragt. Die Herstellung der C₄-Aldehyde erfolgt industriell durch C₃-Hydroformylierung.

Unter Hydroformylierung (Oxo-Reaktion) versteht man allgemein die Umsetzung von ungesättigten Verbindungen wie insbesondere Olefinen (Alkene) mit Synthesegas (Wasserstoff und Kohlenmonoxid) in Aldehyde, deren Anzahl an Kohlenstoffatome um eins höher liegt als die Anzahl der Kohlenstoffatome der Ausgangsverbindungen. Zur Herstellung von C₄-Aldehyden wird dementsprechend Propen hydroformyliert.

Eine gute Übersicht über den Stand der Hydroformylierung von Olefinen findet sich in
B. Cornils, W. A. Herrmann, "Applied Homogeneous Catalysis with Organometallic Compounds", Vol. 1 & 2, VCH, Weinheim, New York, 1996
   sowie in
R. Franke, D. Selent, A. Börner, "Applied Hydroformylation", Chem. Rev., 2012, DOI:10.1021/cr3001803.

Zu den Aldehyden mit fünf Kohlenstoffatomen (kurz: C₅-Aldehyde) gehören n-Pentanal (Valeraldehyd), iso-Pentanal (Isovaleradelhyd), sec-Pentanal (2-Methylbutanal) und tert-Pentanal (Pivalaldehyd). C₅-Aldehyde werden großtechnisch durch Hydroformylierung von C₄-Alkenen hergestellt.

Pentanale dienen unter anderem als Ausgangsstoffe zur Erzeugung von Pentanolen, Pentansäuren und Pentylaminen. Durch Aldolkondensation und Totalhydrierung des Aldolkondensats können aus ihnen Decanole gewonnen werden, die Zwischenprodukte für die Herstellung von Weichmachern, Detergenzien und Schmiermitteln sind. Ein entsprechender Prozess ist in DE102009001594A1 beschrieben.

Da sowohl Butanal als auch Pentanal bedeutende Industriechemikalien darstellen, existiert weltweit eine große Anzahl von Oxo-Anlagen, in welchen entweder eine C₃- oder eine C₄-Hydroformylierung zur Herstellung von Butanal bzw. Pentanal betrieben wird.

Das für diesen Zweck genutzte Propen bzw. Buten fällt in der Regel bei der Raffinierung bzw. beim Cracken von Erdöl an. Die Alkene sind dabei in der Regel keineswegs als Reinstoffe verfügbar sondern stets in Begleitung weiterer Kohlenwasserstoffe anderer Substanzklassen und auch mit mehr oder weniger Kohlenstoffatomen. So wird als Rohstoff für die Propen-Hydroformylierung so genanntes C₃-Gemisch verwendet, welche neben dem ungesättigten C₃-Olefin auch das gesättigte C₃-Alkan Propan enthält. Stärker durchmischt sind so genannte C₄-Gemische, die für die Pentanal-Herstellung genutzt werden: Sie enthalten in wechselnder Zusammensetzung die unterschiedlichen Buten-Isomere sowie die Butane.

Aufgrund der fortschreitenden Verknappung von Erdöl werden zunehmend alternative Quellen für C₃ und C₄-Alkene erschlossen wie etwa Schieferöl oder Schiefergas. Einzelheiten zu schildern ginge hier zu weit; wichtig ist lediglich zu betonen, dass sich die Rohstoffquellen bestehender C₃ und C₄-Oxo-Anlagen verändern und folglich die Bestands-Anlagen mit veränderten Einsatzgemischen betrieben werden müssen. Die Anpassung der Anlagen ist nicht trivial, da die Produktqualität der Aldehyde maßgeblich von der Zusammensetzung der Einsatzgemische bestimmt wird.

Die Erfindung befasst sich im Wesentlichen mit der Frage, wie sich die Wirtschaftlichkeit bestehender Oxo-Prozesse mit technischen Maßnahmen aufrechterhalten lässt, wenn sich die Qualität der Einsatzgemische verschlechtert.

Genauer gesagt befasst sich die Erfindung mit der Nachrüstung von Oxo-Anlagen, die nach dem so genannten "Kreisgasprozess" arbeiten.

Bei einem Kreisgasprozess (engl.: gas recyle; stripping reactor process) werden die Produkte der Hydroformylierung gasförmig mit überschüssigen Synthesegas aus dem Reaktor ausgetragen. Eine allgemeine Schilderung der Hydroformylierung im Kreisgasverfahren findet sich in:
Van Leeuwen, Piet W.N.M und Claver, Carmen (Edit.): Rhodium Catalyzed Hydroformylation. Catalysis by Metal Complexes. Volume 22. Kluwer, 2000, Seiten 212f..

Der Vorteil einer nach dem Kreisgas-Verfahren arbeitende Oxo-Anlage ist ihr einfacher apparativer Aufbau. Deswegen werden zur C₃-Hydroformylierung in der Regel Kreisgas-Anlagen verwendet.

In der C₄-Hydroformylierung sind Kreisgas-Anlagen allerdings weniger weit verbreitet: Aufgrund der vergleichsweise geringen Flüchtigkeit der C₅-Aldehyde und insbesondere der in Nebenreaktionen gebildeten Hochsieder gelten Kreisgasverfahren für die Pentanal-Herstellung als inakzeptabel; vgl. van Leeuwen, a.a.O.

Nichtsdestotrotz hat es Versuche gegeben, C₅-Aldehyde aus C₄-Olefingemischen im Kreisgasverfahren herzustellen:
So beschreibt EP0016285B2 ein Kreisgasverfahren zur Herstellung von Valeraldehyd aus einem C₄-Gemisch enthaltend 2.23 % n-Butan, 1.06 % iso-Butan, 69.88 % 1-Buten, 10.06 % cis-2-Buten und 15.1 % trans-2-Buten.

Ein anderes Verfahren zur C₄-Hydroformylierung gemäß der eingangs genannten Gattung ist aus EP2280920B1 bekannt. Die Erfinder gehen von diesem nächstliegenden Stand der Technik aus.

Bei der in EP2280920B1 praktizierten Kreisgas-Hydroformylierung zur Herstellung von Valeraldehyd wird ein Einsatzgemisch verwendet, welches 35 % 2-Butene enthält und lediglich 1 % 1-Buten. Der Rest ist inertes Butan. Das extrem an 1-Buten arme Gemisch wird mit Hilfe eines symmetrischen Bisphosphit-Liganden hydroformyliert, welcher durch Zugabe eines sterisch gehinderten, sekundären Amins stabilisiert wird. Als Lösungsmittel wird Isononylbenzoat erwähnt. Mit diesem Katalysatorsystem werden Butenumsätze von 60 bis 75 % erzielt. Diese bescheidenen Umsatzwerte gilt es zu steigern.

Eine Möglichkeit zur Steigung des Umsatzes besteht darin, die nicht umgesetzten Olefine in den Hydroformylierungsreaktor zurückzuführen. Dabei ist aber zu beachten, dass nicht zusätzlich inerte Komponenten wie Alkane oder Reaktionsnebenprodukte in den Reaktor zurückgeführt werden, da auf diese Weise die Raum/Zeit-Ausbeute des Prozesses weiter gesenkt wird. Außerdem muss der Kreisgaskompressor eine größere Leistung zur Verdichtung der Inertkomponenten erbringen, wodurch der Energiebedarf des Prozesses steigt.

Eine Möglichkeit, den Umsatz des Kreisgasprozesses zu steigern ohne seine Effizienz zu verschlechtern, besteht darin, das nicht umgesetzte Substrat vor seiner Rückführung in den Reaktor von nicht hydroformylierbaren Stoffen zu reinigen. So beschreibt DE10128325A1 eine homogen katalysierte C₃-Hydroformylierung im Kreisgas-Verfahren, bei welchem aus dem Reaktionsaustrag mit Hilfe einer Membrantrenneinheit ein Permeat gewonnen wird, in welchem sich die im ersten Reaktordurchgang nicht umgesetzten Olefine anreichern. Das Permeat wird in den Kreisgas-Reaktor zurückgeführt und dort erneut in Gegenwart des bereits im ersten Durchgang verwendeten Rhodium-Phosphit-Systems der Hydroformylierung unterzogen. Über das Retentat der Membrantrenneinheit werden inerte Stoffe aus dem Kreisgasprozess ausgeschleust, sodass die Effizienz gesteigert ist.

Geeignete Membranen zur Trennung hydroformylierbarer Alkene von inerten Alkanen sind bekannt. So beschreibt etwa US5062866 eine Membran mit hoher Butan/Buten-Trennwirkung, die gute Dienste in der C₄-Hydroformylierung leisten könnte. Noch besser zur Olefinabtrennung geeignet sind solche Membranen, die mit einem Trägermedium - einem so genannten "Carrier" - infiltriert sind, welcher dazu befähigt ist, mit den Alkenen Verbindungen einzugehen, die durch das trennaktive Membranmaterial schneller permeieren als die Alkane, welche mit dem Carrier keine Verbindungen eingehen. Als Carrier werden Kupfer- oder Silber-Ionen eingesetzt. Eine derartige, für den Langzeitbetrieb geeignete Membran ist in WO2012167362A1 dargestellt.

Mit Hilfe der Alken/Alkan-trennaktiven Membranen lässt sich ein wie in DE10128325A1 beschriebener Kreisgas-Prozess aufbauen, der höhere Umsätze zu erzielen vermag als ein membranloses Kreisgas-Verfahren wie das aus EP2280920B1 bekannte.

Dennoch sind dem Umsatz einer solchen Konstellation Prinzip bedingt weiter Grenzen gesetzt: So erfolgt bei einem Kreisgas-Verfahren mit Permeat-Rezyklierung in den Reaktor die Hydroformylierung zwar in mehreren Durchgängen, jedoch stets in Gegenwart desselben Katalysatorsystems. Die Leistungsfähigkeit des homogenen Katalysatorsystems im Kreisgasreaktor bestimmt somit letztendlich den Umsatz des gesamten Prozesses.

Eine andere Möglichkeit, den Umsatz zu steigern, besteht darin, eine mehrstufige Hydroformylierung vorzusehen. Bei einem mehrstufigen Prozess wird eine Reaktion mehrfach hintereinander ausgeführt, jedoch in jeder Stufe bei anderen Reaktionbedingungen. So existieren zweistufige Hydroformylierungsprozesse, bei denen in der ersten Stufe eine primäre Hydroformylierung in Gegenwart eines Kobalt-Katalysators durchgeführt wird und bei denen in einer nachgeordneten zweiten Stufe die in der ersten Stufe nicht umgesetzten Olefine in Gegenwart eines Rhodium-Systems einer sekundären Hydroformylierung unterworfen werden. Die Mehrstufigkeit ermöglicht es, die Vorteile der jeweiligen Katalysatorsysteme zu kombinieren. So können etwa mit dem sekundären Katalysatorsystem die Bestandteile des Feeds noch umgesetzt werden, welche das Katalysatorsystem der primären Hydroformylierung nicht angreifen kann.

Die Stufen können sich nicht nur hinsichtlich des jeweils eingesetzten Katalysatorsystems unterscheiden, sondern auch durch apparativen Aufbau: So ist aus US4593127A eine zweistufige Hydroformylierung bekannt, deren primäre Hydroformylierung im Kreisgasverfahren betrieben wird und deren sekundäre Hydroformylierung einen flüssigen Produktabzug aufweist. Beide Stufen nutzen einen klassischen Metall/Organophosphor-Komplex als homogenes Katalysatorsystem.

Solche Katalysatorsysteme stellen den Industriestandart dar. Prinzipieller Nachteil homogener Katalysatorsysteme ist jedoch ihre aufwendige Abtrennung, da der Katalysatorkompex im Reaktionssystem gelöst ist.

Deutlich einfacher abzutrennen sind Heterogenkatalysatoren, die als Festkörper praktisch von allein im Reaktor verbleiben. Allerdings sind einfache Festkörperkatalysatoren aufgrund ihrer geringen Aktivität nicht geeignet, eine Hydroformylierung mit der gewünschten Geschwindigkeit zu katalysieren.

Einen Kompromiss stellen so genannte SILP-Systeme dar.

Unter einem SILP (= Supported Ion Liquid Phase) - System versteht man ein Katalysatorsystem, welches einen festes poröses Trägermaterial umfasst, auf dessen Oberfläche eine ionische Flüssigkeit aufgebracht ist. In der ionischen Flüssigkeit gelöst ist wiederum ein homogenes Katalysatorsystem wie zum Beispiel ein Metall/Organophosphor-Komplex. Auf diese Weise wird der Homogenkatalysator über die Ionische Flüssigkeit an dem festen Träger fixiert. Man erhält einen immobilisierten Homogenkatalysator, der sich wie ein Heterogenkatalysator handhaben lässt und die Abtrennung des Katalysators aus dem Reaktionsgemisch signifikant erleichtert. Ein SILP-System kombiniert damit die Vorteile eines aktiven Homogenkatalysators mit denen eines einfach zu handhabenden Heterogenkatalysators. Ein für die C₃- und C₄-Hydroformylierung geeignetes SILP-System ist in WO2012041846A1 offenbart.

Die Durchführbarkeit der SILP-katalysierten Hydroformylierung von Propen und Buten ist von der wissenschaftlichen Literatur belegt.

So zeigen Riisager et al. die Propen-Hydroformylierung an einem SILP-Katalysator umfassend den Liganden 2,7-bis-(SO3Na)-4,5-bis(diphenylphosphino)-9,9-dimethylxanthene in einem modifizierten Rh-Komplex (Rh-Sulfoxantphos):
Riisager, A.; Fehrmann, R.; Haumann, M.; Gorle, B. S. K. & Wasserscheid, P. Stability and Kinetic Studies of Supported lonic Liquid Phase Catalysts for Hydroformylation of Propene Industrial & Engineering Chemistry Research, American Chemical Society, 2005, 44, 9853-9859

Dass sich dasselbe SILP-System auch zur 1-Buten Hydroformylierung eignet, zeigen Haumann et al.:
Haumann, M.; Dentler, K.; Joni, J.; Riisager, A. & Wasserscheid, P. Continuous Gas-Phase Hydroformylation of 1-Butene using Supported lonic Liquid Phase (SILP) Catalysts Adv. Synth. Catal., WILEY-VCH Verlag, 2007, 349, 425-431
Haumann, M.; Jakuttis, M.; Werner, S. & Wasserscheid, P. Supported ionic liquid phase (SILP) catalyzed hydroformylation of 1-butene in a gradient-free loop reactor Journal of Catalysis, 2009, 263, 321-327

Der Erfindung liegt die Aufgabe zu Grunde, das Verfahren der eingangs genannten Gattung dahingehend weiterzubilden, dass es auch bei sich verschlechternder Rohstofflage hohe Umsätze erzielt und Aldehyd in guter Produktqualität liefert. Insbesondere soll eine Lösung gefunden werden, bestehende Oxo-Anlagen für die Verwertung minderwertiger Rohstoffquellen zu ertüchtigen.

Gelöst wird diese Aufgabe dadurch, dass das aldehydarme Gemisch mittels einer Membrantrenneinheit in ein Retentat und in ein Permeat dergestalt aufgetrennt wird, dass im aldehydarmen Gemisch enthaltene Alkene im Permeat angereichert werden währenddessen im aldehydarmen Gemisch enthaltene Alkan im Retentat angereichert werden. Das alkenreiche Permeat wird dann in eine sekundäre Reaktionszone überführt und dort mit Synthesegas in Gegenwart eines SILP-Katalysatorsystems einer sekundären Hydroformylierung unterworfen. Das aus der sekundären Hydroformylierung erhaltene Reaktionsprodukt wird in die Aldehydabtrennung zurückgeführt.

Gegenstand der Erfindung ist mithin ein Verfahren zur Herstellung von Aldehyden durch Hydroformylierung von Alkenen, bei welchem ein alkenhaltiges Einsatzgemisch mit Synthesegas in Gegenwart eines homogenen Katalysatorsystems einer primären Hydroformylierung unterworfen wird, wobei die primäre Hydroformylierung in einer primären Reaktionszone erfolgt, aus welcher kontinuierlich ein Kreisgas enthaltend zumindest ein Teil der Produkte, sowie nicht umgesetzte Edukte der primären Hydroformylierung abgezogen und teilweise kondensiert wird, wobei nicht kondensierte Anteile des Kreisgases in die primäre Reaktionszone zurückgeführt werden, wobei kondensierte Anteile des Kreisgases in einer Aldehydabtrennung destillativ aufgetrennt werden in ein aldehydreiches Gemisch und in ein aldehydarmes Gemisch, wobei das aldehydarme Gemisch mittels einer Membrantrenneinheit in ein Retentat und in ein Permeat dergestalt aufgetrennt wird, dass im aldehydarmen Gemisch enthaltende Alkene im Permeat angereichert werden, währenddessen im aldehydarmen Gemisch enthaltende Alkane im Retentat angereichert werden, bei welchem das Permeat in eine sekundäre Reaktionszone überführt und dort mit Synthesegas in Gegenwart eines SILP-Katalysatorsystems einer sekundären Hydroformylierung unterworfen wird, und bei welchem das aus der sekundären Hydroformylierung erhaltene Reaktionsprodukt der Aldehydabtrennung zugeführt wird.

Die vorliegende Erfindung beruht auf der Erkenntnis, dass die SILP-Technologie in hervorragendem Maße dazu geeignet ist, die übrigen Alkene, die im Kreisgasprozess in Gegenwart eines Organophosphor-Metall-Komplexes nicht umgesetzt wurden, im Wege einer sekundären Hydroformylierung noch in Aldehyd umzusetzen. Die Kombination einer klassisch homogen katalysierten Kreisgas-Stufe mit einer sekundären Hydroformylierungsstufe an einem SILP-System ist deshalb vorteilhaft, weil einer SILP-Stufe die Edukte gasförmig zugeführt werden können. Deshalb ist es mit vergleichsweise geringem technischen Aufwand möglich, die SILP-Stufe an eine Kreisgas-Stufe anzuschließen, indem einfach ein Teil des Kreisgases, wahlweise vor oder nach seiner Teilkondensation, in die SILP-Stufe gefahren wird. Eine Grundidee der Erfindung besteht mithin darin, ein Teil des Kreisgases als Edukt für die SILP-Stufe zu verwenden. Da innerhalb der SILP-Stufe die sekundäre Hydroformylierung praktisch in der Gasphase durchgeführt wird (aus chemischer Sicht findet die Hydroformylierung in einem SILP-System auch in der flüssigen Phase statt, nämlich innerhalb der ionischen Flüssigkeit - aus technischer Sicht ist aber eher die Eduktzuführung und Produktabführung von Interesse; beides erfolgt bei einer SILP-Stufe in der Gasphase) sind zusätzliche Maßnahmen zur Verdampfung des Zulaufs der SILP-Stufe in der Regel verzichtbar, sodass ein kombinierter Kreisgas/SILP-Prozess besonders effizient betrieben werden kann.

Außerdem verringert die SILP-Stufe das Volumen des zirkulierten Kreisgases, da auf Grund des gesteigerten Umsatzes weniger nicht umgesetzte Alkene in den Kreisgasreaktor zurückgeführt werden müssen. Hierdurch sinkt der Energieverbrauch des Kreisgaskompressors. Der Energiebedarf des Kreisgaskompressors verursacht heute etwa 25 % der Gesamtbetriebskosten einer Oxo-Anlage, sodass hier getätigte Einsparungen spürbar sind.

Des Weiteren beruht die Erfindung auf der Erkenntnis, dass der Reaktionsaustrag der SILP-Stufe keine eigene Produktabtrennung benötigt, sondern dass das aus der sekundären Hydroformylierung erhaltene Reaktionsprodukt gemeinsam mit dem Reaktionsprodukt der primären Hydroformylierung in einer gemeinsamen Aldehydabtrennung aufgearbeitet werden kann. Hierdurch werden die Apparatekosten deutlich verringert.

Der besondere Vorteil des erfindungsgemäßen Verfahrens besteht im Übrigen darin, dass die Membrantrenneinheit und der SILP-Reaktor vergleichsweise kleine Apparate darstellen, die einfach an eine bestehende Kreisgas-Oxoanlage und deren Aldehydabtrennung angeschlossen werden kann. So ist es auf diese Weise möglich, aus einem herkömmlichen Verfahren nach dem Oberbegriff des Anspruch 1, welches in einer bestehenden Oxo-Anlage betrieben wird, durch nachträgliche Ergänzung der Membrantrenneinheit und der SILP-Stufe zu einem erfindungsgemäßen, zweistufigen Verfahren zu gelangen, welches einen höheren Umsatz aufweist als das herkömmliche einstufige Verfahren. Mithin können nach der Erfindung bestehende Oxo-Prozesse in einfacher Weise nachgerüstet werden, sobald sich die Rohstoffversorgung der Bestandsanlage verschlechtert.

Das erfindungsgemäße Verfahren kann sowohl in der C₃-Hydroformylierung, als auch in der C₄-Hydroformylierung eingesetzt werden. Grund dafür ist, dass gängige SILP-Systeme sowohl Propen, als auch Buten zu den entsprechenden Aldehyden umsetzen. Aus diesem Grunde kann als Einsatzgemisch für die erste Stufe entweder ein C₃-Gemisch eingesetzt werden, welches zwischen 10 und 90 Gew.-% Alkene mit drei Kohlenstoffatome enthält oder ein C₄-Gemisch eingesetzt werden, welches zwischen 10 und 90 Gew.-% Alkene mit vier Kohlenstoffatomen enthält. Selbstverständlich können auch Einsatzgemische verwendet werden, die sowohl C₃-Olefine als auch C₄-Olefine als hydroformylierbares Substrat enthalten. Nicht ausgeschlossen ist, dass das Einsatzgemisch weitere Alkene enthält, die weniger als drei oder mehr als vier Kohlenstoffatome aufweisen. Bevorzugt sind freilich solche Gemische, die besonders sortenrein sind. In der betrieblichen Praxis werden jedoch eher heterogene Gemische verarbeitet werden müssen, deren Zusammensetzung sogar schwankt.

Der besondere Charme der Erfindung besteht darin, dass die sekundäre SILP-Hydroformylierung sich gleichermaßen für die Erweiterungen von C₃-Prozessen, als auch für die Erweiterung von C₄-Prozessen eignet. Dies an einem Standort, an dem sowohl eine C₃-Hydroformylierung, als auch eine C₄-Hydroformylierung ausgeübt wird, eröffnet sich die Möglichkeit, lediglich eine einzige SILP-Stufe nachzurüsten, die wahlweise der C₃-Oxoanlage oder der C₄-Oxoanlage zugeordnet wird. In einer solchen Konstellation wird parallel aus dem C₃-Gemisch C₄-Aldehyde und aus dem C₄-Gemisch C₅-Aldehyde hergestellt unter der Maßgabe,
dass das C₃-Gemisch mit Synthesegas in Gegenwart eines homogenen Katalysatorsystems einer primären C₃-Hydroformylierung unterworfen wird, wobei die primäre C₃-Hydroformylierung in einer primären C₃-Reaktionszone erfolgt, aus welcher kontinuierlich ein C₃-Kreisgas enthaltend zumindest ein Teil der Produkte sowie nicht umgesetzte Edukte der primären C₃-Hydroformylierung abgezogen, teilkondensiert und nicht kondensierte Anteile des C₃-Kreisgases in die primäre C₃-Reaktionszone zurückgeführt werden, und wobei die kondensierten Anteile des C₃-Kreisgases in einer C₄-Aldehydabtrennung destillativ aufgetrennt werden in ein C₄-aldehydreiches Gemisch und in ein C₄-aldehydarmes Gemisch,
und dass das C₄-Gemisch mit Synthesegas in Gegenwart eines homogenen Katalysatorsystems einer primären C₄-Hydroformylierung unterworfen wird, wobei die primäre C₄-Hydroformylierung in einer primären C₄-Reaktionszone erfolgt, aus welcher kontinuierlich ein C₄-Kreisgas enthaltend zumindest ein Teil der Produkte sowie nicht umgesetzte Edukte der primären C₄-Hydroformylierung abgezogen, teilkondensiert und nicht kondensierte Anteile des C₄-Kreisgases in die primäre C₄-Reaktionszone zurückgeführt werden, und wobei die kondensierten Anteile des C₄-Kreisgases in einer C₅-Aldehydabtrennung destillativ aufgetrennt werden in ein C₅-aldehydreiches Gemisch und in ein C₅-aldehydarmes Gemisch,
wobei wahlweise das C₄-aldehydarme Gemisch oder das C₅-aldehydarme Gemisch der Membrantrenneinheit zugeführt und das erhaltene Permeat der sekundären Hydroformylierung in Gegenwart des SILP-Katalysatorsystems unterworfen wird,
und wobei das aus der sekundären Hydroformylierung erhaltene Reaktionsprodukt der entsprechenden C₄- bzw. C₅-Aldehydabtrennung zugeführt wird.

Der Vorteil dieser wahlweise Zuordnung der SILP-Stufe zu der C₃- bzw. zu der C₄-Anlage ist insbesondere dann vorteilhaft, wenn sowohl das gelieferte C₃-Gemisch, als auch das gelieferte C₄-Gemisch qualitativen Schwankungen unterliegen die unterschiedlich stark ausgeprägt sind. Je nachdem, welche Rohstoffquelle momentan die schlechtere Qualität liefert, wird die SILP-Stufe der jeweiligen diesen Rohstoffstrom verwertenden Oxo-Anlage zugeordnet. Selbstverständlich kann die SILP-Stufe auch der Oxo-Anlage zugeordnet werden, deren Aldehyde augenblicklich in größeren Mengen nachgefragt wird.

Auf diese Weise hält man durch eine überschaubare Neuinstallation von einer Membranabtrennung und einer SILP-Stufe neben den beiden Oxo-Anlagen eine erhebliche Steigerung in der Produktions- und Rohstoffflexibilität.

Maßgeblich für den Erfolg des erfindungsgemäßen Verfahrens ist, dass mittels des Membrantrennverfahrens der aldehydarme Strom aus der Aldehydabtrennung möglichst vollständig von Inertkomponenten abgereichert wird, die der SILP-Stufe nicht als hydroformylierbares Substrat zur Verfügung stehen. Hierzu gehören insbesondere die Alkane, welche sich auf Grund ihres nahezu identischen Molekulargewichts sich kaum von den entsprechenden Olefinen destillativ trennen lassen. Eine besonders effektive Abtrennung der nicht hydroformylierbaren Alkane kann mit Hilfe einer Membrantrenneinheit erfolgen, die mindestens eine Membran mit einem trennaktiven Membranmaterial umfasst, wobei die Membrantrenneinheit mit einem Trägermedium versehen ist, welches dazu befähigt ist mit Alkenen Verbindungen einzugehen, für welche das Membranmaterial eine höhere Permeabilität aufweist als für die entsprechenden nicht verbundenen Alkene. Unter dem Trägermedium ist ein Carrier zu verstehen, der sich mit dem im Permeat anzureichernden Alkenen verbindet zu Verbindungen, die bevorzugt durch das Membranmaterial permeieren. Auf diese Weise wird die Trennschärfe der Membran in Richtung der Alkene deutlich gesteigert. Mit den reaktionsträgeren Alkanen geht das Trägermedium bzw. der Carrier keine Verbindung ein, so dass die Alkane deutlich langsamer permeieren und deswegen bevorzugt im Retentat angereichert werden. Als Trägermedium eignen sich insbesondere Kupfer- oder Silber-Ionen.

Eine geeignete Membran mit hoher Butan/Buten Trennwirkung wird in US5062866 beschrieben.

Ho et al. beschreiben eine Supported Liquid Membran", in der die Flüssigkeit eine Silberlösung mit einer hohen präferenzielle Löslichkeit für Olefine ist:
W.S. Ho, D.C. Dalrymple, Facilitated transport of olefins in Ag+-containing polymer membranes, Journal of Membrane Science 91 (1994) 13-25.

Auch eine solche Membran ist für den hier beabsichtigen Zweck gut geeignet. Dies gilt umso mehr, wenn die von Ho et al. beschriebene Supported Liquid Membrane unmittelbar im Brüdenstrom der zur Aldehydabtrennung genutzten Kolonne, also noch stromaufwärts vor dem Kopfkondensator der Kolonne angeordnet wird:
Hierdurch wird keine zusätzliche Kondensation notwendig und der Brüdenstrom, der kondensiert wird und flüssig auf die Kolonne gegeben wird, ist aufgrund der Abtrennung des Permeats deutlich reduziert. Dies spart Energiekosten durch Reduktion des Rücklaufstroms. Außerdem liefert der Kopfdruck der Kolonne nach Ho et al. einen besseren C₄-Olefinfluss über die Membran, bei nur sehr leichter Abnahme des Trennfaktores C₄-Olefine gegenüber C₄-Alkanen. Aufgrund einer anschließenden Hydroformylierung in der Gasphase in einem SILP-Reaktor muss der Permeatstrom nicht so hoch verdichtet werden, wie in einer klassischen Flüssigphasenhydroformylierung und spart deswegen zusätzliche Energiekosten gegenüber dem klassischen Prozess ein. Der Kolonnenrücklauf wird dann aus dem Retentat der Membran gespeist.

Weitere geeignete Membranen mit Olefin/Paraffin-Trennwirkung sind offenbart in
R. Faiz, K. Li, Olefin/paraffin separation using membrane based facilitated transport/chemical absorption techniques, Chemical Engineering Science 73 (2012) 261-284.
R. Faiz, K. Li, Polymeric membranes for light olefin/paraffin separation, Desalination 287 (2012) 82-97.

Eine kommerziell verfügbare Membran bzw. System für Olefin/Paraffin Trennung mittels Membranverfahren wird von der Fa. IMTEX Membranes Corp., Mississauga, Canada angeboten. IMTEX beschreibt ein Verfahren zum Langzeitbetrieb einer Olefin/Paraffin selektiven Membran in WO2012/167362A1.

Das SILP-Katalysatorsystem der sekundären Hydroformylierung umfasst vorzugsweise die folgenden Komponenten:
a) eine festes poröses Trägermaterial;
b) eine ionische Flüssigkeit;
c) ein Metall ausgewählt aus der 9. Gruppe des Periodensystems der Elemente;
d) einen phosphorhaltigen organischen Liganden;
e) optional ein organisches Amin.

Ein solches SILP-System ist in WO2012041846A1 beschreiben.

Vorzugsweise tritt das Permeat der Membrantrennstufe gasförmig in die sekundäre Reaktionszone ein. Hierdurch wird der Energieverbrauch aufgrund geringerer Verdichterleistungen gegenüber einer klassischen Flüssigphasenhydroformylierung reduziert. Dies gilt umso mehr, wenn das das Permeat gasförmig in der Membrantrenneinheit anfällt. Dies wird dann der Fall sein, wenn der über die Membran gemessene Transmembrandruck so groß ist, dass Permeat aufgrund des Druckverlustes praktisch an der Membran verdampft (Gaspermeation). Dann können weitere Maßnahmen zur Verdampfung des Permeats vor Eintritt in die SILP-Stufe entfallen.

Fällt das Permeat in der Membrantrenneinheit zumindest teilweise flüssig an, ist indes ein Verdampfer notwendig, welcher vermittels Wärmeeinwirkung das flüssige Permeat vor dem Eintritt in die SILP-Stufe vollständig verdampft. Die hierfür erforderliche Wärme kann beispielsweise aus dem aus dem Kondensator gewonnen werden, der in der primären Hydroformylierung zur Teilkondensierung des Kreisgases genutzt wird. Auf diese Weise wird Energie eingespart.

Als Katalysatorsystem in dem Kreisgasprozess wird vorzugsweise ein klassisches Rhodium-/Phosphin- oder Phosphit-oder Phosphoramidit-System genutzt, welches vollständig in der flüssigen Phase des Reaktionsgemisches der primären Reaktionszone gelöst ist.

Gegenstand der Erfindung ist auch eine Anlage zur Herstellung von Aldehyden durch Hydroformylierung von Alkenen umfassend eine Membrantrenneinheit und eine im Permeat der Membrantrenneinheit angeordnete sekundäre Reaktionszone, in welcher ein SILP-Katalysatorsystem gegenwärtig ist.

Diese Anlage umfasst die Baugruppen, um welche eine bestehende Oxo-Anlage ergänzt werden muss, um das erfindungsgemäße Verfahren durchführen zu können.

Vorzugsweise weist die Membrantrenneinheit der Anlage ein Trägermedium/Carrier auf zur Steigerung der Alken-Selektivität und der Membran.

Das SILP-Katalysatorsystem der Anlage weist vorzugsweise die oben genannten Merkmale a) bis d) und optional auch e) auf.

Gegenstand der Erfindung ist auch ein Anlagenkomplex zur Durchführung eines erfindungsgemäßen Verfahrens unter Verwendung der erfindungsgemäßen Erweiterung der Anlage. Ein solcher Anlagenkomplex setzt sich dann aus einem ersten Teilkomplex für die Durchführung der primären Hydroformylierung, sowie aus einem zweiten Teilkomplex zusammen, in welchem die sekundäre Hydroformylierung durchgeführt wird. Der erste Teilkomplex entspricht dann der Bestands-Oxo-Anlage, währenddessen der zweite Komplex die erfindungsgemäße Erweiterung darstellt. Erster und zweiter Teilkomplex sind so miteinander zu verschalten, dass die Membrantrenneinheit des zweiten Teilkomplexes mit mindestens einem aldehydhaltigen Stoffstrom aus dem ersten Teilkomplex beaufschlagbar ist.

Wie bereits geschildert, lässt sich die erfindungsgemäße Erweiterung mit unterschiedlichen, parallel betriebenen Oxo-Anlagen kombinieren. Auf diese Weise sieht eine bevorzugte Weiterbildung der Erfindung vor, dass ein Anlagenkomplex bestehend aus drei Teilkomplexen betrieben wird, wobei in dem ersten und in dem dritten Teilkomplex eine primäre Hydroformylierung durchgeführt wird, währenddessen in dem zweiten Teilkomplex die SILP-basierte Hydroformylierung durchgeführt wird. Die SILP-Stufe ist dann wahlweise mit einem aldehydhaltigen Stoffstrom aus dem ersten Teilkomplex und/oder aus dem dritten Teilkomplex beaufschlagbar.

Sofern die beiden sekundären Hydroformylierungen unterschiedliche Aldehyde produzieren, werden sie auch eine eigene Aldehyd-Abtrennung aufweisen, die auf die Abtrennung des allerdings mit der entsprechenden Kohlenstoffanzahl eingerichtet ist. Die Rückführung des Reaktionsprodukts aus der sekundären Reaktionszone des zweiten Teilkomplexes folgt dementsprechend in die Aldehydabtrennung des ersten bzw. des dritten Teilkomplexes, je nachdem, aus welchem Teilkomplex der der sekundären Hydroformylierung zugeführte aldehydarme Stoffstrom stammt: Grundsätzlich wird der Reaktionsaustrag der SILP-Stufe in die Aldehydabtrennung zurückgeführt, aus der der Feed der SILP-Stufe letztendlich stammt.

Wie bereits oben ausgeführt, besteht eine Grundidee der Erfindung darin, ein Teil des Kreisgases als Edukt für die SILP-Stufe zu verwenden. Der Teil des Kreisgases, der als Edukt auf die SILP-Stufe gefahren wird, kann wahlweise vor oder nach der Teilkondensation aus dem Kreisgasprozess entnommen werden.

Bisher wurden die Ausführungsformen der Erfindung geschildert, bei der die SILP-Stufe hinter der Teilkondensation angeordnet ist und praktisch aus den kondensierten Anteilen des Kreisgases gespeist wird.

Erfindungsgemäß ist es aber auch möglich, die zweite Reaktionszone noch vor der Teilkondensation anzuordnen und sie direkt mit aus der ersten Reaktionszone abgezogenem Kreisgas zu speisen. Der Auslauf der SILP-Stufe wird dann der Teilkondensation zugeführt, sodass die Aufarbeitung des SILP-Austrags letztendlich über dieselbe Kolonne erfolgt, die auch die Aldehydabtrennung der Kreisgasstufe besorgt.

Gegenstand der Erfindung ist mithin auch ein Verfahren zur Herstellung von Aldehyden durch Hydroformylierung von Alkenen, bei welchem ein alkenhaltiges Einsatzgemisch mit Synthesegas in Gegenwart eines homogenen Katalysatorsystems einer primären Hydroformylierung unterworfen wird, wobei die primäre Hydroformylierung in einer primären Reaktionszone erfolgt, aus welcher kontinuierlich ein Kreisgas enthaltend zumindest ein Teil der Produkte, sowie nicht umgesetzte Edukte der primären Hydroformylierung abgezogen und teilweise kondensiert wird, wobei nicht kondensierte Anteile des Kreisgases in die primäre Reaktionszone zurückgeführt werden, und wobei kondensierte Anteile des Kreisgases in einer Aldehydabtrennung destillativ aufgetrennt werden in ein aldehydreiches Gemisch und in ein aldehydarmes Gemisch, bei welchem das Kreisgas vor seiner Teilkondensation mittels einer Membrantrenneinheit in ein Retentat und in ein Permeat dergestalt aufgetrennt wird, wobei im Kreisgas enthaltende Alkene im Permeat angereichert werden, währenddessen im Kreisgas enthaltende Alkane im Retentat angereichert werden, und wobei das Permeat in eine sekundäre Reaktionszone überführt und dort mit Synthesegas in Gegenwart eines SILP-Katalysator¬systems einer sekundären Hydroformylierung unterworfen wird, und wobei das aus der sekundären Hydroformylierung erhaltene Reaktionsprodukt der Teilkondensation zugeführt wird.

Wie bei der anderen Anordnungsvariante der Membrantrenneinheit wird auch bei Beaufschlagung der Membrantrenneinheit mit noch nicht kondensiertem Kreisgas eine Membrantrenneinheit mit einem trennaktiven Membranmaterial eingesetzt, welche mit einem Trägermedium versehen ist, welches dazu befähigt ist, mit Alkenen Verbindungen einzugehen, für welche das Membranmaterial eine höhere Permeabilität aufweist als für die entsprechenden nicht verbundenen Alkene.

Die SILP-System der zweiten Hydroformylierungsstufe umfasst auch bei dieser Variante die vorerörterten Komponenten a) bis d) und optional auch e).

Bei dem Katalysatorsystem in der primären Hydroformylierung (Kreisgas-Stufe) wird vorzugsweise ein klassisches homogenes Katalysatorsystem umfassend Rhodium und mindestens einen Phosphin- oder Phosphit-oder Phosphoramidit-Liganden eingesetzt, wobei das homogene Katalysatorsystem vollständig in einer flüssigen Phase des Reaktionsgemisches der primären Reaktionszone gelöst ist.

Der besondere Vorteil des hier beschriebenen Nachrüstsatzes umfassend im Wesentlichen die Membrantrenneinheit und die SILP-Stufe besteht darin, dass er gleichwohl vor und hinter der Teilkondensation des Kreisgases angeordnet werden kann.

Gegenstand der Erfindung ist mithin auch ein Anlagenkomplex für die Durchführung eines Verfahrens, bei dem die SILP-Stufe vor der Teilkondensation angeordnet ist, unter Verwendung des universell einsetzbaren Nachrüstsatzes, sodass der Anlagenkomplex einen ersten Teilkomplex für die Durchführung einer primären Hydroformylierung und einen zweiten Teilkomplex für die Durchführung einer sekundären Hydroformylierung aufweist, wobei die Membrantrenneinheit des zweiten Teilkomplexes mit dem Kreisgas aus dem ersten Teilkomplex beaufschlagbar ist.

Da die besonderen Vorteile der Erfindung insbesondere im Wege einer Nachrüstung einer bestehenden Anlage erzielt werden, ist die Verwendung einer Anlage umfassend Membranabtrennung und SILP-Hydroformylierung zur Nachrüstung einer bestehenden Anlage zur homogen katalysierten Hydroformylierung, die insbesondere nach dem Kreisgasverfahren arbeitet, ebenfalls Gegenstand der Erfindung.

Selbstverständlich kann eine erfindungsgemäße, zweistufig arbeitende Oxo-Anlage auch als komplette Neuanlage konzipiert werden.

Die Erfindung soll nun anhand von Ausführungsbeispielen erläutert werden. Hierfür zeigen:
- Figur 1:: vereinfachtes Prozessfließbild eines zweistufigen Oxo-Prozesses gemäß der Erfindung, bei dem die SILP-Stufe hinter der Teilkondensation des Kreisgases angeordnet ist;
- Figur 2:: vereinfachtes Prozessfließbild eines Anlagenkomplexes, der zwei primäre Hydroformylierungen aufweist;
- Figur 3:: vereinfachtes Prozessfließbild eines zweistufigen Oxo-Prozesses gemäß der Erfindung, bei dem die SILP-Stufe vor der Teilkondensation des Kreisgases angeordnet ist.

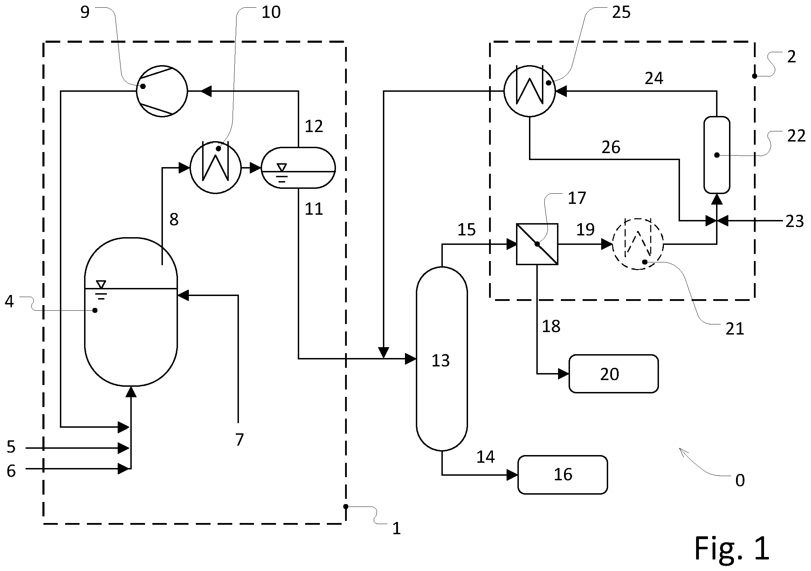

Figur 1 zeigt ein vereinfachtes Prozessfließbild zur Durchführung einer zweistufen Hydroformylierung. Der dafür notwendige Anlagenkomplex 0 setzt sich zusammen aus einem ersten Teilkomplex 1 und einem zweiten Teilkomplex 2. Der erste Teilkomplex 1 dient zur Durchführung einer primären Hydroformylierung zum Beispiel von Butenen im Kreisgasverfahren, währenddessen der zweite Teilkomplex 2 für die Umsetzung der im ersten Teilkomplex 1 nicht umgesetzten Butene im Wege einer sekundären SILP-Hydroformylierung bestimmt ist.

Der erste Teilkomplex 1 entspricht einer herkömmlichen Kreisgas-Anlage für die Buten-Hydroformylierung, wie sie beispielsweise aus EP2280920B1 bekannt ist.

Herzstück der Kreisgas-Anlage ist eine primäre Reaktionszone 4 zum Beispiel in Gestalt eines Blasensäulen-Reaktors eines Rührkessels, eines Schlaufenreaktors oder eines Strahldüsenreaktors. Innerhalb der primären Reaktionszone 4 bilden sich eine flüssige Reaktionsphase und eine gasförmige Reaktionsphase aus. Die flüssige Phase wird dabei im Wesentlichen von flüssigem Reaktionsprodukt (C₅-Aldehyd), gelöstem Synthesegas und Butenen, sowie von gelöstem Homogenkatalysator gebildet. Des Weiteren können flüssige Lösungsmittel, wie beispielsweise Isononylbenzoat enthalten sein.

Ein die zu hydroformylierenden Butene umfassendes C₄-Gemisch 5 und Synthesegas 6, bestehend aus Kohlenmonoxid und Wasserstoff, werden in die primäre Reaktionszone 4 hineingefahren. Soweit erforderlich, wird frischer Katalysator 7 ebenfalls in die primäre Reaktionszone 4 hineingefahren. Bei dem Katalysator 7 handelt es sich insbesondere um ein herkömmliches Rhodium/Phosphit-System.

In an sich bekannter Weise wird innerhalb der primären Reaktionszone 4 das C₄-Gemisch 5 mit Synthesegas 6 in Gegenwart des homogen gelösten Katalysators 7 in entsprechende C₅-Aldehyde umgesetzt. Die C₅-Aldehyde werden zusammen mit überschüssigem Synthesegas aus der Gasphase der primären Reaktionszone 4 als Kreisgas 8 abgezogen. Das Ansaugen des Kreisgases 8 erfolgt über einen KreisgasKompressor 9. In einer Wärmetauscher-Kondensator-Kombination 10 erfolgt eine Teilkondensation des Kreisgases 8. Dabei fällt ein Kondensat 11 an, welches im Wesentlichen die C₅-Aldehyde, als auch nicht umgesetzte Butene und inerte Bestandteile des C₄-Gemisches 5 umfasst. Die nicht kondensierten Bestandteile des Kreisgases 12 umfassen im Wesentlichen nicht umgesetztes Synthesegas. Sie werden zurück in die primäre Reaktionszone 4, also in den Kreisgasreaktor gefahren.

Das Kondensat 11 wird indessen in eine Aldehydabtrennung 13 verbracht. Die Aldehydabtrennung 13 arbeitet im Wesentlichen destillativ. So wird das Kondensat 11 aufgetrennt in ein aldehydreiches Gemisch 14 und ein aldehydarmes Gemisch 15.

Das aldehydreiche Gemisch 14 wird zu einer Aldolisierung 16 geleitet, um dort in an sich bekannter Weise einer Aldolkondensation unterworfen zu werden. Dieser Prozess ist in DE102009001594A1 beschrieben.

Das aldehydarme Gemisch 15, bevorzugt der nicht kondensierte Brüden der als Aldehydabtrennung genutzten Destillationskolonne, wird indes in den zweiten Teilkomplex 2 überführt. Dort wird es zunächst einer Membrantrenneinheit 17 zugeführt und darin aufgetrennt in ein Retentat 18 und ein Permeat 19. Die Membrantrenneinheit 17 kann in an sich bekannter Weise eine oder mehrere Membranmodule umfassen die miteinander parallel oder seriell verschaltet sind. Auf die genaue Ausgestaltung der Membrantrenneinheit kommt es hier nicht an. Entscheidend ist vielmehr, dass die Membrantrenneinheit 17 geeignet ist, die im aldehydarmen Gemisch 15 enthaltenen Olefine in ihrem Permeat 19 anzureichern, währenddessen die inerten Alkane im Retentat 18 angereichert werden. Geeignete Membranen sind im oben zitierten Stand der Technik beschrieben.

Die im aldehydarmen Gemisch 15 enthaltenen Butane stammen ursprünglich aus dem C₄-Gemisch 5 und werden zusätzlich in einer hydrierenden Nebenreaktion innerhalb der primären Reaktionszone 4 aus Butenen und Wasserstoff gebildet. Die Butane sind der Hydroformylierung nicht ohne weiteres mehr zugänglich, so dass sie über das Retentat 18 der Membrantrenneinheit ausgeschleust und einer Butanverwertung 20 zugeführt werden. Die hier nicht näher beschriebene Butanverwertung 20 umfasst im Wesentlichen eine Hydrierung der verbleibenden ungesättigten Verbindungen sowie eine Aufreinigung. Das so erhaltene Butan kann als Treibstoff oder als Brennstoff genutzt werden.

Sollte die Membrantrenneinheit 17 direkt aus dem Brüden der Aldehydabtrennung 13 gespeist werden, wird ein Teil des Retentats dem nicht dargestellten Kopfkondensator als Aldehydabtrennung genutzten Destillationskolonne zugeführt. Der abgezweigte Teil des Retentats entspricht dann dem Kolonnenrücklauf.

Die im ersten Durchgang innerhalb der primären Reaktionszone 4 nicht hydroformylierten Alkene sammeln sich letztendlich im Permeat 19 der Membrantrenneinheit 17 an. Sofern der Transmembrandruck groß genug ist, verdampft das Permeat praktisch beim Austritt aus der Membran. Sollte das Permeat 19 noch nicht vollständig verdampft sein, kann es über einen optionalen Verdampfer 21 verdampft werden. Der Verdampfer 21 wird mit Wärme aus dem Wärmetauscher 10 betrieben, um Energie einzusparen. Spätestens hinter dem Verdampfer 21 ist das Permeat 19 gasförmig. In diesem Zustand wird es in eine sekundäre Reaktionszone 22 überführt und darin an einem SILP-Katalysatorsystem einer sekundären Hydroformylierung unterworfen. Die sekundäre Hydroformylierung benötigt auch Synthesegas. Sofern dieses im Permeat 19 nicht ausreichend vorhanden ist, wird zusätzliches Synthesegas 23 zudosiert. Die sekundäre Hydroformylierung in der sekundären Reaktionszone 22 erfolgt in Gegenwart eines SILP-Katalysatorsystems in an sich bekannter Weise wie in WO2012041846A1 beschrieben. Der Reaktionsaustrag 24 der sekundären Reaktionszone 22 ist ebenfalls gasförmig. Es wird in einem Kühler 25 gekühlt wobei es sich wieder teilweise verflüssigt. Dabei ausgasendes Synthesegas 26 kann in die sekundäre Reaktionszone 22 rezykliert werden. Der gekühlte Reaktionsaustrag 24 der sekundären Hydroformylierung wird schließlich zurück in die Aldehydabtrennung 13 geführt. Die in der SILP-Hydroformylierung gebildeten C₅-Aldehyde werden mithin in derselben Aldehydabtrennung 13 abgetrennt wie die Pentanale, die in der primären Hydroformylierung im ersten Teilkomplex 1 gebildet werden. Die zweite Stufe im zweiten Teilkomplex 2 kommt somit ohne eigene Aldehydabtrennung aus.

Gemäß einer bevorzugten Weiterbildung der Erfindung handelt es sich bei dem zweiten Teilkomplex 2 umfassend im Wesentlichen die Membrantrenneinheit 17 und die sekundäre Reaktionszone 22 um eine Erweiterung einer bestehenden Kreisgasanlage 1.

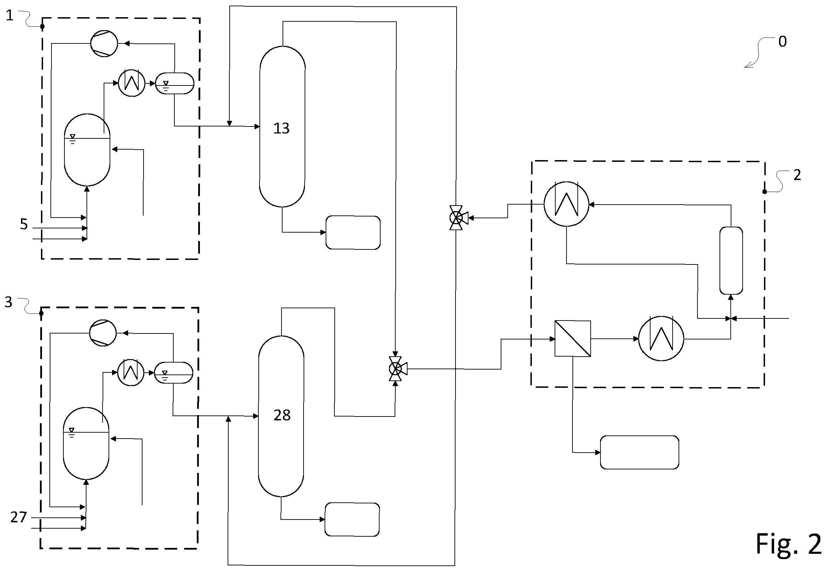

Einen besonderen Vorteil lässt sich mit der Erfindung erzielen, wenn innerhalb eines Verbundstandortes eine C₄-Hydroformylierung 1 neben einer C₃-Hydroformylierung 3 betrieben wird. Nach einer bevorzugten Weiterbildung der Erfindung ist es möglich, die C₃-Oxoanlage 3 und die C₄-Oxoanlage 1 um eine SILP-Hydroformylierung 2 zu ergänzen, die wahlweise mit der C₄-Oxoanlage 1 und der C₃-Oxoanlage 3 kombinierbar ist. Auf diese Weise erhält man den in Figur 2 dargestellten Anlagenkomplex 0 umfassen drei Teilkomplexe 1, 2, 3.

Die Abhängigkeit der Produktqualität des für den ersten Teilkomplex eingesetzten C₄-Gemisches 5 und des für den im dritten Teilkomplex 3 eingesetzten C₃-Gemisches 27 wird der SILP-Komplex 2 wahlweise mit der C₄-Oxoanlage 1 oder mit der C₃-Oxoanlage 3 gekoppelt. Die Rückführung aus dem SILP-Prozess erfolgt dann in die C₅-Aldehydabtrennung 13 bzw. die C₄-Aldehydabtrennung 28.

Auf diese Weise kann mit dem dreiteiligen Anlagenkomplex aus Figur 2 auf unterschiedliche Produktqualitäten der bereitgestellten Gemische 5, 27 bzw. der jeweiligen Nachfrage nach Butanal bzw. Pentanal reagiert werden.

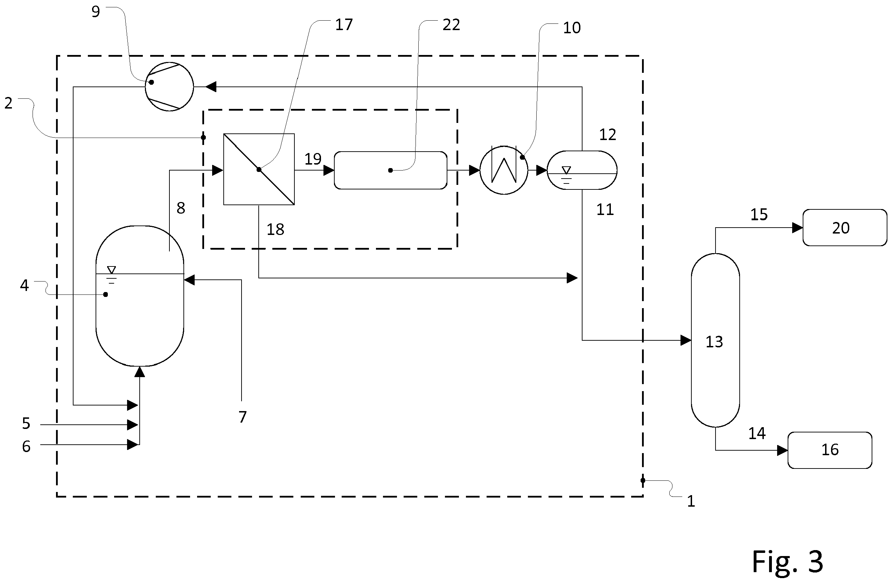

In der Figur 3 ist eine weitere Ausführungsform der Erfindung dargestellt, bei der zweite Teilkomplex 2 innerhalb des ersten Teilkomplexes 1 angeordnet ist, nämlich noch vor der Wärmetauscher/Kondensator-Kombination 10, in welcher die Teilkondensation des Kreisgases 8 erfolgt. Die Membrantrenneinheit 17 wird direkt mit dem frisch aus der primären Hydroformylierung abgezogenen Kreisgas 8 beaufschlagt. Im Kreisgas 8 enthaltene Alkene werden im Permeat 19 der Membrantrenneinheit 17 angereichert und der SILP-basierten, sekundären Reaktionszone 22 zugeführt um dort umgesetzt zu werden. Die nicht hydroformylierbaren Alkane sammeln sich bevorzugt im Retentat 18 der Membrantrenneinheit 17 und werden Richtung Aldehydabtrennung 13 aus dem Kreisgas ausgeschleust. Dadurch wird der Kreisgaskompressor 9 entlastet.

Der Reaktionsaustrag 24 der sekundären Hydroformylierung 22 wird der Wärmetauscher/Kondensator-Kombination 10 zugeführt, sodass die Aufarbeitung des Austrags beider Hydroformylierungen 4, 22 gemeinsam in der Aldehydabtrennung 13 erfolgt.

Vorteil dieser Ausgestaltung der Erfindung gegenüber der in Figur 1 dargestellten Variante ist, dass bis zu zwei Wärmetauscher eingespart werden können. Nachteil ist der größere Volumenstrom durch die SILP-Stufe, sodass die Membrantrenneinheit 17 und die sekundäre Reaktionszone 22 entsprechend größer dimensioniert werden müssen. Letztendlich entscheidet eine Wirtschaftlichkeitsbetrachtung über die bevorzugte Verfahrensvariante.

### Bezugszeichenliste

- 0: Anlagenkomplex
- 1: erster Teilkomplex (C₄-Oxoanlage)
- 2: zweiter Teilkomplex (SILP-Anlage)
- 3: dritter Teilkomplex (C₃-Oxoanlage)
- 4: primäre Reaktionszone
- 5: C₄-Gemisch
- 6: Synthesegas
- 7: Katalysator
- 8: Kreisgas
- 9: Kreisgaskompressor
- 10: Wärmetauscher/Kondensator-Kombination
- 11: Kondensat
- 12: nicht kondensierte Bestandteile des Kreisgases
- 13: (C₅-)Aldehydabtrennung
- 14: aldehydreiches Gemisch
- 15: aldehydarmes Gemisch
- 16: Aldolkondensation
- 17: Membrantrenneinheit
- 18: Retentat
- 19: Permeat
- 20: Butanverwertung
- 21: Verdampfer
- 22: Sekundäre Reaktionszone
- 23: zusätzliches Synthesegas
- 24: Reaktionsaustrag der sekundären Hydroformylierung
- 25: Kühler
- 26: ausgasendes Synthesegas
- 27: C₃-Gemisch
- 28: C₄-Aldehydabtrennung

## Patentansprüche

1. Verfahren zur Herstellung von Aldehyden durch Hydroformylierung von Alkenen, bei welchem ein alkenhaltiges Einsatzgemisch mit Synthesegas in Gegenwart eines homogenen Katalysatorsystems einer primären Hydroformylierung unterworfen wird, wobei die primäre Hydroformylierung in einer primären Reaktionszone erfolgt, aus welcher kontinuierlich ein Kreisgas enthaltend zumindest ein Teil der Produkte, sowie nicht umgesetzte Edukte der primären Hydroformylierung abgezogen und teilweise kondensiert wird, wobei nicht kondensierte Anteile des Kreisgases in die primäre Reaktionszone zurückgeführt werden, und wobei kondensierte Anteile des Kreisgases in einer Aldehydabtrennung destillativ aufgetrennt werden in ein aldehydreiches Gemisch und in ein aldehydarmes Gemisch,
**dadurch gekennzeichnet,**
**dass** das aldehydarme Gemisch mittels einer Membrantrenneinheit in ein Retentat und in ein Permeat dergestalt aufgetrennt wird, dass im aldehydarmen Gemisch enthaltende Alkene im Permeat angereichert werden, währenddessen im aldehydarmen Gemisch enthaltende Alkane im Retentat angereichert werden, und dass das Permeat in eine sekundäre Reaktionszone überführt und dort mit Synthesegas in Gegenwart eines SILP-Katalysatorsystems einer sekundären Hydroformylierung unterworfen wird, wobei das aus der sekundären Hydroformylierung erhaltene Reaktionsprodukt der Aldehydabtrennung zugeführt wird.

2. Verfahren nach Anspruch 1,
**dadurch gekennzeichnet,**
**dass** als Einsatzgemisch ein C₃-Gemisch eingesetzt wird, welches zwischen 10 und 90 Gew.-% Alkene mit drei Kohlenstoffatomen enthält, bezogen auf das Gesamtgewicht des Einsatzgemisches.

3. Verfahren nach Anspruch 1,
**dadurch gekennzeichnet,**
**dass** als Einsatzgemisch ein C₄-Gemisch eingesetzt wird, welches zwischen 10 und 90 Gew.-% Alkene mit vier Kohlenstoffatomen enthält, bezogen auf das Gesamtgewicht des Einsatzgemisches.

4. Verfahren nach Anspruch 2 und 3, bei welchem parallel aus dem C₃-Gemisch C₄-Aldehyde und aus den C₄-Gemisch C₅-Aldehyde hergestellt werden, unter der Maßgabe
dass das C₃-Gemisch mit Synthesegas in Gegenwart eines homogenen Katalysatorsystems einer primären C₃-Hydroformylierung unterworfen wird, wobei die primäre C₃-Hydroformylierung in einer primären C₃-Reaktionszone erfolgt, aus welcher kontinuierlich ein C₃-Kreisgas enthaltend zumindest ein Teil der Produkte sowie nicht umgesetzte Edukte der primären C₃-Hydroformylierung abgezogen, teilkondensiert und nicht kondensierte Anteile des C₃-Kreisgases in die primäre C₃-Reaktionszone zurückgeführt werden, und wobei die kondensierten Anteile des C₃-Kreisgases in einer C₄-Aldehydabtrennung destillativ aufgetrennt werden in ein C₄-aldehydreiches Gemisch und in ein C₄-aldehydarmes Gemisch,
und dass das C₄-Gemisch mit Synthesegas in Gegenwart eines homogenen Katalysatorsystems einer primären C₄-Hydroformylierung unterworfen wird, wobei die primäre C₄-Hydroformylierung in einer primären C₄-Reaktionszone erfolgt, aus welcher kontinuierlich ein C₄-Kreisgas enthaltend zumindest ein Teil der Produkte sowie nicht umgesetzte Edukte der primären C₄-Hydroformylierung abgezogen, teilkondensiert und nicht kondensierte Anteile des C₄-Kreisgases in die primäre C₄-Reaktionszone zurückgeführt werden, und wobei die kondensierten Anteile des C₄-Kreisgases in einer C₅-Aldehydabtrennung destillativ aufgetrennt werden in ein C₅-aldehydreiches Gemisch und in ein C₅-aldehydarmes Gemisch,
wobei wahlweise das C₄-aldehydarme Gemisch oder das C₅-aldehydarme Gemisch der Membrantrenneinheit zugeführt und das erhaltene Permeat der sekundären Hydroformylierung in Gegenwart des SILP-Katalysatorsystems unterworfen wird,
und wobei das aus der sekundären Hydroformylierung erhaltene Reaktionsprodukt der entsprechenden C₄- bzw. C₅-Aldehydabtrennung zugeführt wird.

5. Verfahren nach einem der vorhergehenden Ansprüche, wobei die Membrantrenneinheit mindestens eine Membran mit einem trennaktiven Membranmaterial umfasst,
**dadurch gekennzeichnet,**
**dass** die Membrantrenneinheit mit einem Trägermedium versehen ist, welches dazu befähigt ist, mit Alkenen Verbindungen einzugehen, für welche das Membranmaterial eine höhere Permeabilität aufweist als für die entsprechenden nicht verbundenen Alkene.

6. Verfahren nach einem der vorhergehenden Ansprüche,
**dadurch gekennzeichnet,**
**dass** das SILP-Katalysatorsystem der sekundären Hydroformylierung die folgenden Komponenten umfasst:
a) eine festes poröses Trägermaterial;
b) eine ionische Flüssigkeit;
c) ein Metall ausgewählt aus der 9. Gruppe des Periodensystems der Elemente;
d) einen phosphorhaltigen organischen Liganden;
e) optional ein organisches Amin.

7. Verfahren nach einem der Ansprüche 1 bis 6,
**dadurch gekennzeichnet,**
**dass** das Permeat gasförmig in die sekundäre Reaktionszone eintritt.

8. Verfahren nach Anspruch 7,
**dadurch gekennzeichnet,**
**dass** das Permeat zumindest teilweise flüssig in der Membrantrenneinheit anfällt und vor Eintritt in die sekundäre Reaktionsstufe durch einen Verdampfer vermittels Wärmeeinwirkung verdampft wird.

9. Verfahren nach Anspruch 7,
**dadurch gekennzeichnet,**
**dass** das Permeat gasförmig in der Membrantrenneinheit anfällt.

10. Verfahren nach einem der vorhergehenden Ansprüche,
**dadurch gekennzeichnet,**
**dass** das homogene Katalysatorsystem der primären Hydroformylierung Rhodium und mindestens einen Phosphin- oder Phosphit-oder Phosphoramidit-Liganden umfasst, wobei das homogene Katalysatorsystem vollständig in einer flüssigen Phase des Reaktionsgemisches der primären Reaktionszone gelöst ist.

11. Anlage zur Herstellung von Aldehyden durch Hydroformylierung von Alkenen umfassend eine Membrantrenneinheit und eine im Permeat der Membrantrenneinheit angeordnete sekundäre Reaktionszone, in welcher ein SILP-Katalysatorsystem gegenwärtig ist.

12. Anlage nach Anspruch 10, wobei die Membrantrenneinheit mindestens eine Membran mit einem trennaktiven Membranmaterial umfasst,
**dadurch gekennzeichnet,**
**dass** die Membrantrenneinheit mit einem Trägermedium versehen ist, welches dazu befähigt ist, mit Alkenen Verbindungen einzugehen, für welche das Membranmaterial eine höhere Permeabilität aufweist als für die entsprechenden nicht verbundenen Alkene.

13. Anlage nach Anspruch 11 oder 12,
**dadurch gekennzeichnet,**
**dass** das SILP-Katalysatorsystem die folgenden Komponenten umfasst:
a) eine festes poröses Trägermaterial;
b) eine ionische Flüssigkeit;
c) ein Metall ausgewählt aus der 9. Gruppe des Periodensystems der Elemente;
d) einen phosphorhaltigen organischen Liganden;
e) optional ein organisches Amin.

14. Anlagenkomplex für die Durchführung eines Verfahrens nach einem der Ansprüche 1 bis 10 unter Verwendung einer Anlage nach Anspruch 11, 12 oder 13, umfassend mindestens einen ersten Teilkomplex für die Durchführung einer primären Hydroformylierung sowie eine Anlage nach Anspruch 11, 12 oder 13 als zweiter Teilkomplex für die Durchführung einer sekundären Hydroformylierung, wobei die Membrantrenneinheit des zweiten Teilkomplexes mit mindestens einem aldehydhaltigen Stoffstrom aus dem ersten Teilkomplex beaufschlagbar ist.

15. Anlagenkomplex nach Anspruch 14 für die Durchführung eines Verfahrens nach Anspruch 4 unter Verwendung einer Anlage nach Anspruch 11, 12 oder 13, weiter umfassend einen dritten Teilkomplex für die Durchführung einer zweiten primären Hydroformylierung,
**dadurch gekennzeichnet,**
**dass** die Membrantrenneinheit des zweiten Teilkomplexes wahlweise mit dem aldehydhaltigen Stoffstrom aus dem ersten Teilkomplex und/oder mit einem aldehydhaltigen Stoffstrom aus dem dritten Teilkomplex beaufschlagbar ist.

16. Anlagenkomplex nach einem der Ansprüche 14 oder 15, **gekennzeichnet durch** eine Rückführung mittels welcher ein Reaktionsprodukt der sekundären Reaktionszone in eine Aldehydabtrennung des ersten und/oder dritten Teilkomplexes rückführbar ist.

17. Verfahren zur Herstellung von Aldehyden durch Hydroformylierung von Alkenen, bei welchem ein alkenhaltiges Einsatzgemisch mit Synthesegas in Gegenwart eines homogenen Katalysatorsystems einer primären Hydroformylierung unterworfen wird, wobei die primäre Hydroformylierung in einer primären Reaktionszone erfolgt, aus welcher kontinuierlich ein Kreisgas enthaltend zumindest ein Teil der Produkte, sowie nicht umgesetzte Edukte der primären Hydroformylierung abgezogen und teilweise kondensiert wird, wobei nicht kondensierte Anteile des Kreisgases in die primäre Reaktionszone zurückgeführt werden, und wobei kondensierte Anteile des Kreisgases in einer Aldehydabtrennung destillativ aufgetrennt werden in ein aldehydreiches Gemisch und in ein aldehydarmes Gemisch,
**dadurch gekennzeichnet,**
**dass** das Kreisgas vor seiner Teilkondensation mittels einer Membrantrenneinheit in ein Retentat und in ein Permeat dergestalt aufgetrennt wird, dass im Kreisgas enthaltende Alkene im Permeat angereichert werden, währenddessen im Kreisgas enthaltende Alkane im Retentat angereichert werden, und dass das Permeat in eine sekundäre Reaktionszone überführt und dort mit Synthesegas in Gegenwart eines SILP-Katalysatorsystems einer sekundären Hydroformylierung unterworfen wird, wobei das aus der sekundären Hydroformylierung erhaltene Reaktionsprodukt der Teilkondensation zugeführt wird.

18. Verfahren nach Anspruch 17, wobei die Membrantrenneinheit mindestens eine Membran mit einem trennaktiven Membranmaterial umfasst,
**dadurch gekennzeichnet,**
**dass** die Membrantrenneinheit mit einem Trägermedium versehen ist, welches dazu befähigt ist, mit Alkenen Verbindungen einzugehen, für welche das Membranmaterial eine höhere Permeabilität aufweist als für die entsprechenden nicht verbundenen Alkene.

19. Verfahren nach Anspruch 17 oder 18,
**dadurch gekennzeichnet,**
**dass** das SILP-Katalysatorsystem der sekundären Hydroformylierung die folgenden Komponenten umfasst:
a) eine festes poröses Trägermaterial;
b) eine ionische Flüssigkeit;
c) ein Metall ausgewählt aus der 9. Gruppe des Periodensystems der Elemente;
d) einen phosphorhaltigen organischen Liganden;
e) optional ein organisches Amin.

20. Verfahren nach Anspruch 17, 18 oder 19,
**dadurch gekennzeichnet,**
**dass** das homogene Katalysatorsystem der primären Hydroformylierung Rhodium und mindestens einen Phosphin- oder Phosphit-oder Phosphoramidit-Liganden umfasst, wobei das homogene Katalysatorsystem vollständig in einer flüssigen Phase des Reaktionsgemisches der primären Reaktionszone gelöst ist.

21. Anlagenkomplex für die Durchführung eines Verfahrens nach einem der Ansprüche 17 bis 20 unter Verwendung einer Anlage nach Anspruch 11, 12 oder 13, umfassend mindestens einen ersten Teilkomplex für die Durchführung einer primären Hydroformylierung sowie eine Anlage nach Anspruch 11, 12 oder 13 als zweiter Teilkomplex für die Durchführung einer sekundären Hydroformylierung, wobei die Membrantrenneinheit des zweiten Teilkomplexes mit dem Kreisgas aus dem ersten Teilkomplex beaufschlagbar ist.

22. Verwendung einer Anlage nach Anspruch 11, 12 oder 13 zur Nachrüstung einer bestehenden Anlage zur homogen katalysierten Hydroformylierung.

## Claims

1. Process for preparing aldehydes by hydroformylation of alkenes, in which an alkene-containing feed mixture is subjected to a primary hydroformylation with synthesis gas in the presence of a homogeneous catalyst system, the primary hydroformylation being effected in a primary reaction zone from which a cycle gas containing at least some of the products and unconverted reactants of the primary hydroformylation are drawn off continuously and partly condensed, with recycling of uncondensed components of the cycle gas into the primary reaction zone, and with distillative separation of condensed components of the cycle gas in an aldehyde removal stage to give an aldehyde-rich mixture and a low-aldehyde mixture,
**characterized in that**
the low-aldehyde mixture is separated into a retentate and a permeate by means of a membrane separation unit in such a way that alkenes present in the low-aldehyde mixture become enriched in the permeate, while alkanes present in the low-aldehyde mixture become enriched in the retentate, and **in that** the permeate is transferred into a secondary reaction zone and subjected to a secondary hydroformylation therein with synthesis gas in the presence of an SILP catalyst system, with supply of the reaction product obtained from the secondary hydroformylation to the aldehyde removal stage.

2. Process according to Claim 1,
**characterized in that**
the feed mixture used is a C₃ mixture containing between 10% and 90% by weight of alkenes having three carbon atoms, based on the total weight of the feed mixture.

3. Process according to Claim 1,
**characterized in that**
the feed mixture used is a C₄ mixture containing between 10% and 90% by weight of alkenes having four carbon atoms, based on the total weight of the feed mixture.

4. Process according to Claims 2 and 3, in which there is parallel preparation of C₄ aldehydes from the C₃ mixture and C₅ aldehydes from the C₄ mixture, with the proviso
that the C₃ mixture is subjected to a primary C₃ hydroformylation with synthesis gas in the presence of a homogeneous catalyst system, the primary C₃ hydroformylation being effected in a primary C₃ reaction zone from which a C₃ cycle gas containing at least some of the products and unconverted reactants of the primary C₃ hydroformylation are drawn off continuously and partly condensed, and uncondensed components of the C₃ cycle gas being recycled into the primary C₃ reaction zone, and the condensed components of the C₃ cycle gas being separated by distillation in a C₄ aldehyde removal stage to give a C₄ aldehyde-rich mixture and a low-C₄ aldehyde mixture,
and in that the C₄ mixture is subjected to a primary C₄ hydroformylation with synthesis gas in the presence of a homogeneous catalyst system, the primary C₄ hydroformylation being effected in a primary C₄ reaction zone from which a C₄ cycle gas containing at least some of the products and unconverted reactants of the primary C₄ hydroformylation are drawn off continuously and partly condensed, and uncondensed components of the C₄ cycle gas being recycled into the primary C₄ reaction zone, and the condensed components of the C₄ cycle gas being separated by distillation in a C₅ aldehyde removal stage to give a C₅ aldehyde-rich mixture and a low-C₅ aldehyde mixture,
wherein, optionally, the low-C₄ aldehyde mixture or the low-C₅ aldehyde mixture is fed to the membrane separation unit and the resultant permeate is subjected to the secondary hydroformylation in the presence of the SILP catalyst system,
and wherein the reaction product obtained from the secondary hydroformylation is fed to the corresponding C₄ or C₅ aldehyde removal stage.

5. Process according to any of the preceding claims, wherein the membrane separation unit comprises at least one membrane having a separation-active membrane material,
**characterized in that**
the membrane separation unit has been provided with a carrier medium capable of entering into compounds with alkenes for which the membrane material has a higher permeability than for the corresponding non-compounded alkenes.

6. Process according to any of the preceding claims,
**characterized in that**
the SILP catalyst system of the secondary hydroformylation comprises the following components:
a) a solid porous carrier material;
b) an ionic liquid;
c) a metal selected from group 9 of the Periodic Table of the Elements;
d) a phosphorus-containing organic ligand;
e) optionally an organic amine.

7. Process according to any of Claims 1 to 6,
**characterized in that**
the permeate enters the secondary reaction zone in gaseous form.

8. Process according to Claim 7,
**characterized in that**
the permeate is obtained at least partly in liquid form in the membrane separation unit and, prior to entry into the secondary reaction stage, is evaporated by the action of heat by means of an evaporator.

9. Process according to Claim 7,
**characterized in that**
the permeate is obtained in gaseous form in the membrane separation unit.

10. Process according to any of the preceding claims,
**characterized in that**
the homogeneous catalyst system of the primary hydroformylation comprises rhodium and at least one phosphine or phosphite or phosphoramidite ligand, the homogeneous catalyst system being fully dissolved in a liquid phase of the reaction mixture of the primary reaction zone.

11. Plant for preparation of aldehydes by hydroformylation of alkenes, comprising a membrane separation unit and a secondary reaction zone disposed in the permeate of the membrane separation unit and with an SILP catalyst system present therein.

12. Plant according to Claim 10, wherein the membrane separation unit comprises at least one membrane having a separation-active membrane material,
**characterized in that**
the membrane separation unit has been provided with a carrier medium capable of entering into compounds with alkenes for which the membrane material has a higher permeability than for the corresponding non-compounded alkenes.

13. Plant according to Claim 11 or 12,
**characterized in that**
the SILP catalyst system comprises the following components:
a) a solid porous carrier material;
b) an ionic liquid;
c) a metal selected from group 9 of the Periodic Table of the Elements;
d) a phosphorus-containing organic ligand;
e) optionally an organic amine.

14. Plant complex for the performance of a process according to any of Claims 1 to 10 using a plant according to Claim 11, 12 or 13, comprising at least one first complex component for the performance of a primary hydroformylation and a plant according to Claim 11, 12 or 13 as a second complex component for the performance of a secondary hydroformylation, wherein the membrane separation unit of the second complex component can be charged with at least one aldehyde-containing stream from the first complex component.

15. Plant complex according to Claim 14 for the performance of a process according to Claim 4 using a plant according to Claim 11, 12 or 13, further comprising a third complex component for the performance of a second primary hydroformylation,
**characterized in that**
the membrane separation unit of the second complex component can be charged either with the aldehyde-containing stream from the first complex component and/or with an aldehyde-containing stream from the third complex component.

16. Plant complex according to either of Claims 14 and 15, **characterized by** recycling by means of which a reaction product from the secondary reaction zone can be recycled into an aldehyde removal stage in the first and/or third complex component.

17. Process for preparing aldehydes by hydroformylation of alkenes, in which an alkene-containing feed mixture is subjected to a primary hydroformylation with synthesis gas in the presence of a homogeneous catalyst system, the primary hydroformylation being effected in a primary reaction zone from which a cycle gas containing at least some of the products and unconverted reactants of the primary hydroformylation are drawn off continuously and partly condensed, with recycling of uncondensed components of the cycle gas into the primary reaction zone, and with distillative separation of condensed components of the cycle gas in an aldehyde removal stage to give an aldehyde-rich mixture and a low-aldehyde mixture,
**characterized in that**
the cycle gas, prior to the partial condensation thereof, is separated into a retentate and a permeate by means of a membrane separation unit in such a way that alkenes present in the cycle gas become enriched in the permeate, while alkanes present in the cycle gas become enriched in the retentate, and **in that** the permeate is transferred into a secondary reaction zone and subjected to a secondary hydroformylation therein with synthesis gas in the presence of an SILP catalyst system, with supply of the reaction product obtained from the secondary hydroformylation to the partial condensation stage.

18. Process according to Claim 17, wherein the membrane separation unit comprises at least one membrane having a separation-active membrane material,
**characterized in that**
the membrane separation unit has been provided with a carrier medium capable of entering into compounds with alkenes for which the membrane material has a higher permeability than for the corresponding non-compounded alkenes.

19. Process according to Claim 17 or 18,
**characterized in that**
the SILP catalyst system of the secondary hydroformylation comprises the following components:
a) a solid porous carrier material;
b) an ionic liquid;
c) a metal selected from group 9 of the Periodic Table of the Elements;
d) a phosphorus-containing organic ligand;
e) optionally an organic amine.

20. Process according to Claim 17, 18 or 19,
**characterized in that**
the homogeneous catalyst system of the primary hydroformylation comprises rhodium and at least one phosphine or phosphite or phosphoramidite ligand, the homogeneous catalyst system being fully dissolved in a liquid phase of the reaction mixture of the primary reaction zone.

21. Plant complex for the performance of a process according to any of Claims 17 to 20 using a plant according to Claim 11, 12 or 13, comprising at least one first complex component for the performance of a primary hydroformylation and a plant according to Claim 11, 12 or 13 as a second complex component for the performance of a secondary hydroformylation, wherein the membrane separation unit of the second complex component can be charged with the cycle gas from the first complex component.

22. Use of a plant according to Claim 11, 12 or 13 for retrofitting of an existing plant for homogeneously catalysed hydroformylation.

## Revendications

1. Procédé de fabrication d'aldéhydes par hydroformylation d'alcènes, selon lequel un mélange de départ contenant des alcènes est soumis à une hydroformylation primaire avec un gaz de synthèse en présence d'un système catalytique homogène, l'hydroformylation primaire ayant lieu dans une zone de réaction primaire, à partir de laquelle un gaz circulaire contenant au moins une partie des produits, ainsi que des réactifs non réagis de l'hydroformylation primaire, est soutiré en continu et partiellement condensé, les fractions non condensées du gaz circulaire étant recyclées dans la zone de réaction primaire et les fractions condensés du gaz circulaire étant séparées par distillation dans une séparation des aldéhydes en un mélange riche en aldéhydes et en un mélange pauvre en aldéhydes,
**caractérisé en ce que** le mélange pauvre en aldéhyde est séparé au moyen d'une unité de séparation à membrane en un rétentat et en un perméat, de sorte que les alcènes contenus dans le mélange pauvre en aldéhydes s'accumulent dans le perméat, tandis que les alcanes contenus dans le mélange pauvre en aldéhydes s'accumulent dans le rétentat, et **en ce que** le perméat est transféré dans une zone de réaction secondaire, et y est soumis à une hydroformylation secondaire avec un gaz de synthèse en présence d'un système catalytique SILP, le produit de réaction obtenu à partir de l'hydroformylation secondaire étant introduit dans la séparation des aldéhydes.

2. Procédé selon la revendication 1, **caractérisé en ce qu'**un mélange en C₃ est utilisé en tant que mélange de départ, qui contient entre 10 et 90 % en poids d'alcènes à trois atomes de carbone, par rapport au poids total du mélange de départ.

3. Procédé selon la revendication 1, **caractérisé en ce qu'**un mélange en C₄ est utilisé en tant que mélange de départ, qui contient entre 10 et 90 % en poids d'alcènes à quatre atomes de carbone, par rapport au poids total du mélange de départ.

4. Procédé selon les revendications 2 et 3, selon lequel des aldéhydes en C₄ à partir du mélange en C₃ et des aldéhydes en C₅ à partir du mélange en C₄ sont fabriqués en parallèle, à condition que
le mélange en C₃ soit soumis à une hydroformylation primaire des C₃ avec un gaz de synthèse en présence d'un système catalytique homogène, l'hydroformylation primaire des C₃ ayant lieu dans une zone de réaction primaire des C₃, à partir de laquelle un gaz circulaire en C₃ contenant au moins une partie des produits, ainsi que des réactifs non réagis de l'hydroformylation primaire des C₃, est soutiré en continu, partiellement condensé, et les fractions non condensées du gaz circulaire en C₃ sont recyclées dans la zone de réaction primaire des C₃, et les fractions condensées du gaz circulaire en C₃ sont séparées par distillation dans une séparation des aldéhydes en C₄ en un mélange riche en aldéhydes en C₄ et en un mélange pauvre en aldéhydes en C₄,
et que le mélange en C₄ soit soumis à une hydroformylation primaire des C₄ avec un gaz de synthèse en présence d'un système catalytique homogène, l'hydroformylation primaire des C₄ ayant lieu dans une zone de réaction primaire des C₄, à partir de laquelle un gaz circulaire en C₄ contenant au moins une partie des produits, ainsi que des réactifs non réagis de l'hydroformylation primaire des C₄, est soutiré en continu, partiellement condensé, et les fractions non condensées du gaz circulaire en C₄ sont recyclées dans la zone de réaction primaire des C₄, et les fractions condensées du gaz circulaire en C₄ sont séparées par distillation dans une séparation des aldéhydes en C₅ en un mélange riche en aldéhydes en C₅ et en un mélange pauvre en aldéhydes en C₅,
le mélange pauvre en aldéhydes en C₄ ou le mélange pauvre en aldéhydes en C₅ étant introduit dans l'unité de séparation à membrane, et le perméat obtenu étant soumis à l'hydroformylation secondaire en présence du système catalytique SILP,
et le produit de réaction obtenu à partir de l'hydroformylation secondaire étant introduit dans la séparation des aldéhydes en C₄ ou C₅ correspondante.

5. Procédé selon l'une quelconque des revendications précédentes, dans laquelle l'unité de séparation à membrane comprend au moins une membrane comprenant un matériau de membrane à action de séparation, **caractérisé en ce que** l'unité de séparation à membrane est munie d'un matériau support qui est apte à former avec les alcènes des composés pour lesquels le matériau de membrane présente une perméabilité plus élevée que pour les alcènes non reliés correspondants.

6. Procédé selon l'une quelconque des revendications précédentes, **caractérisé en ce que** le système catalytique SILP de l'hydroformylation secondaire comprend les composants suivants :
a) un matériau support poreux solide ;
b) un liquide ionique ;
c) un métal choisi dans le 9^{e} groupe du tableau périodique des éléments ;
d) un ligand organique contenant du phosphore ;
e) éventuellement une amine organique.

7. Procédé selon l'une quelconque des revendications 1 à 6, **caractérisé en ce que** le perméat entre sous forme gazeuse dans la zone de réaction secondaire.

8. Procédé selon la revendication 7, **caractérisé en ce que** le perméat se forme au moins en partie sous forme liquide dans l'unité de séparation à membrane et est évaporé avant l'entrée dans l'étape de réaction secondaire par un évaporateur sous l'effet de chaleur.

9. Procédé selon la revendication 7, **caractérisé en ce que** le perméat se forme sous forme gazeuse dans l'unité de séparation à membrane.

10. Procédé selon l'une quelconque des revendications précédentes, **caractérisé en ce que** le système catalytique homogène de l'hydroformylation primaire comprend du rhodium et au moins un ligand phosphine ou phosphite ou phosphoramidite, le système catalytique homogène étant dissous en totalité dans une phase liquide du mélange réactionnel de la zone de réaction primaire.

11. Unité pour la fabrication d'aldéhydes par hydroformylation d'alcènes, comprenant une unité de séparation à membrane et une zone de réaction secondaire agencée dans le perméat de l'unité de séparation à membrane, dans laquelle un système catalytique SILP est présent.

12. Unité selon la revendication 10, dans laquelle l'unité de séparation à membrane comprend au moins une membrane comprenant un matériau de membrane à action de séparation, **caractérisée en ce que** l'unité de séparation à membrane est munie d'un matériau support qui est apte à former avec les alcènes des composés pour lesquels le matériau de membrane présente une perméabilité plus élevée que pour les alcènes non reliés correspondants.

13. Unité selon la revendication 11 ou 12, **caractérisée en ce que** le système catalytique SILP comprend les composants suivants :
a) un matériau support poreux solide ;
b) un liquide ionique ;
c) un métal choisi dans le 9^{e} groupe du tableau périodique des éléments ;
d) un ligand organique contenant du phosphore ;
e) éventuellement une amine organique.

14. Complexe d'unités pour la réalisation d'un procédé selon l'une quelconque des revendications 1 à 10, utilisant une unité selon la revendication 11, 12 ou 13, comprenant au moins un premier complexe partiel pour la réalisation d'une hydroformylation primaire, ainsi qu'une unité selon la revendication 11, 12 ou 13 en tant que deuxième complexe partiel pour la réalisation d'une hydroformylation secondaire, l'unité de séparation à membrane du deuxième complexe partiel pouvant être chargée avec au moins un courant de matière contenant des aldéhydes issu du premier complexe partiel.

15. Complexe d'unités selon la revendication 14 pour la réalisation d'un procédé selon la revendication 4, utilisant une unité selon la revendication 11, 12 ou 13, comprenant en outre un troisième complexe partiel pour la réalisation d'une deuxième hydroformylation primaire, **caractérisé en ce que** l'unité de séparation à membrane du deuxième complexe partiel peut être chargée au choix avec le courant de matière contenant des aldéhydes issu du premier complexe partiel et/ou avec un courant de matière contenant des aldéhydes issu du troisième complexe partiel.

16. Complexe d'unités selon l'une quelconque des revendications 14 ou 15, **caractérisé par** un recyclage au moyen duquel un produit de réaction de la zone de réaction secondaire peut être recyclé dans une séparation des aldéhydes du premier et/ou troisième complexe partiel.

17. Procédé de fabrication d'aldéhydes par hydroformylation d'alcènes, selon lequel un mélange de départ contenant des alcènes est soumis à une hydroformylation primaire avec un gaz de synthèse en présence d'un système catalytique homogène, l'hydroformylation primaire ayant lieu dans une zone de réaction primaire à partir de laquelle un gaz circulaire contenant au moins une partie des produits, ainsi que des réactifs non réagis de l'hydroformylation primaire, est soutiré en continu et partiellement condensé, les fractions non condensées du gaz circulaire étant recyclées dans la zone de réaction primaire et les fractions condensés du gaz circulaire étant séparées par distillation dans une séparation des aldéhydes en un mélange riche en aldéhydes et en un mélange pauvre en aldéhydes,
**caractérisé en ce que** le gaz circulaire est séparé avant sa condensation partielle au moyen d'une unité de séparation à membrane en un rétentat et en un perméat, de sorte que les alcènes contenus dans le gaz circulaire s'accumulent dans le perméat, tandis que les alcanes contenus dans le gaz circulaire s'accumulent dans le rétentat, et **en ce que** le perméat est transféré dans une zone de réaction secondaire et y est soumis à une hydroformylation secondaire avec un gaz de synthèse en présence d'un système catalytique SILP, le produit de réaction obtenu à partir de l'hydroformylation secondaire étant introduit dans la condensation partielle.

18. Procédé selon la revendication 17, dans lequel l'unité de séparation à membrane comprend au moins une membrane comprenant un matériau de membrane à action de séparation, **caractérisé en ce que** l'unité de séparation à membrane est munie d'un matériau support qui est apte à former avec les alcènes des composés pour lesquels le matériau de membrane présente une perméabilité plus élevée que pour les alcènes non reliés correspondants.

19. Procédé selon la revendication 17 ou 18, **caractérisé en ce que** le système catalytique SILP de l'hydroformylation secondaire comprend les composants suivants :
a) un matériau support poreux solide ;
b) un liquide ionique ;
c) un métal choisi dans le 9^{e} groupe du tableau périodique des éléments ;
d) un ligand organique contenant du phosphore ;
e) éventuellement une amine organique.

20. Procédé selon la revendication 17, 18 ou 19, **caractérisé en ce que** le système catalytique homogène de l'hydroformylation primaire comprend du rhodium et au moins un ligand phosphine ou phosphite ou phosphoramidite, le système catalytique homogène étant dissous en totalité dans une phase liquide du mélange réactionnel de la zone de réaction primaire.

21. Complexe d'unités pour la réalisation d'un procédé selon l'une quelconque des revendications 17 à 20, utilisant une unité selon la revendication 11, 12 ou 13, comprenant au moins un premier complexe partiel pour la réalisation d'une hydroformylation primaire, ainsi qu'une unité selon la revendication 11, 12 ou 13 en tant que deuxième complexe partiel pour la réalisation d'une hydroformylation secondaire, l'unité de séparation à membrane du deuxième complexe partiel pouvant être chargée avec le gaz circulaire issu du premier complexe partiel.

22. Utilisation d'une unité selon la revendication 11, 12 ou 13 pour la mise à niveau d'une unité existante pour l'hydroformylation sous catalyse homogène.
